(19) Europäisches Patentamt
European Patent Office
Office européen des brevets

(11) Publication number: **0 365 158 B1**

(12) # EUROPEAN PATENT SPECIFICATION

(45) Date of publication of patent specification: **16.02.94**

(51) Int. Cl.5: **C12N 9/06**, C12P 13/06, C12N 1/20, //(C12N1/20, C12R1:01)

(21) Application number: **89309840.0**

(22) Date of filing: **27.09.89**

(54) **Process for production of l-alanine dehydrogenase and a microorganism strain of the genus sporolactobacillus.**

(30) Priority: **27.09.88 JP 241537/88**

(43) Date of publication of application:
**25.04.90 Bulletin 90/17**

(45) Publication of the grant of the patent:
**16.02.94 Bulletin 94/07**

(84) Designated Contracting States:
**DE FR IT**

(56) References cited:
**US-A- 3 262 862**

**PATENT ABSTRACTS OF JAPAN, vol. 10, no. 28 (C-326)[2085], 4th February 1986 & JP-A-60180590**

**TRENDS IN BIOTECHNOLOGY, vol. 7, no. 8, August 1989, pages 210-214, Elsevier**

**Science Publishers Ltd. (UK), Cambridge, GB; T. OHSHIMA et al.: "Thermostableamino acid dehydrogenases: applications and gene cloning"**

(73) Proprietor: **ASAHI KASEI KOGYO KABUSHIKI KAISHA**
**2-6, Dojimahama 1-chome**
**Kita-ku**
**Osaka(JP)**

(72) Inventor: **Takahashi, Mamoru**
**684-1, Kakita**
**Shimizu-cho**
**Sunto-gun Shizuoka(JP)**
Inventor: **Nagasawa, Shinobu**
**296, Mamiya**
**Kannami-cho**
**Tagata-gun Shizuoka(JP)**
Inventor: **Ikuta, Shigeru**
**1277-5, Kashiya**
**Kannami-cho**
**Tagata-gun Shizuoka(JP)**
Inventor: **Matsuura, Kazuo**
**1005-5, Yokka-machi**
**Nirayama-cho**
**Tagata-gun(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

(74) Representative: **Woods, Geoffrey Corlett et al**
**J.A. KEMP & CO.**
**14 South Square**
**Gray's Inn**
**London WC1R 5EU (GB)**

## Description

This invention relates to a process for the production of L-alanine dehydrogenase and to a microorganism strain belonging to the genus Sporolactobacillus.

L-alanine dehydrogenase is an enzyme which catalysess the reaction (I);

L-alanine + $H_2O$ + NAD⇆pyruvate + $NH_4^+$ + NADH + $H^+$

L-alanine dehydrogenase has been used for the quantitative measurement of pyruvate or ammonium ions directly by measuring the decrease of NADH, and has also been used for the production of L-alanine (JP-A-60/184393 and JP-A-62/36196. Hitherto known processes for the production of alanine are those using a microorganism belonging to the genus Thermus (JP-A-58/212782 and JP-A-59/55186), to the genus Bacillus (JP-A-60/180580 and JP-A-60/18090) and to the genus Streptomyces (JP-A-59/132889).

The present invention provides a process for the production of L-alanine dehydrogenase, which comprises culturing a L-alanine dehydrogenase-producing microorganism belonging to genus Sporolactobacillus in a nutrient medium and isolating L-alanine dehydrogenase from the cultured mass.

The present invention also provides a microorganism strain belonging to the genus Sporolactobacillus which is a thermophile which cannot grow at 40°C and can grow at 45°C and 52°C.

The present invention further provides a culture of a strain as defined above in a culture medium containing an assimilable source of carbon, an assimilable source of nitrogen and, if desired, vitamins and inorganic salts other than any such salts which may be used as a nitrogen source, and substantially free from other microorganisms.

The microorganism strain belonging to the genus Sporolactobacillus has been isolated from a soil sample of a hot spring in Kumamoto Pref., Japan.

Taxonomical properties of the microorganism strain belonging to the genus Sporolactobacillus are as follows.

A. Morphological properties :

① Form and arrangement :
Round edge with straight or slightly curved bacillus and singlar or double linked chain slightly short chain.
② Size :
0.6~ 0.8 × 2.0 ~ 4.0 µm.
③ Mobility :
Peritrichal movement.
④ Spore :
Spore formation, but cells not bulged.
⑤ Polymorphism :
Appears Tadpole-like cells at 15 ~ 20 hours culture and forms mixture of rod-shaped cells and spherical cells.

B. Macroscopic observation :

① Nutrient agar slant medium :
Growth : weak, filiform.
Color : transparent, ocherous.
Soluble pigment : no production.
② Nutrient agar plate medium :
Growth : weak, round and flat ~ convex smooth and small colony.
Color : transparent, ocherous.
Soluble pigment : no production.
③ Liquid medium :
Weak growth, uniformly turbid.
④ BCP milk medium :
No change.

C. Physiological and biochemical properties :

〔+: positive, −: negative, (+) weakly positive 〕

| | | |
|---|---|---|
| Gram-stain | | + |
| KOH reaction | | − |
| Acid fastness stain | | − |
| Capsule formation | | − |
| OF-test ( Hugh Leifson ) | | no change |
| Aerobic growth | | (+) |
| Anaerobic growth | | − |
| Growth temperature | 52℃ | + |
| | 45℃ | + |
| | 40℃ | − |
| Halotolerant NaCℓ conc. % | | |
| | 0 % | + |
| | 2.0 % | + |
| | 3.5 % | − |
| Growth pH | pH 9.8 | − |
| | pH 9.0 | + |
| | pH 5.2 | + |
| | pH 4.1 | − |
| Catalase production | | − |
| Oxidase production | | − |
| Urease production | | − |

| | |
|---|---|
| Gelatin hydrolysis | no change |
| Starch hydrolysis | − |
| Casein hydrolysis | no change |
| Esculin hydrolysis | + |
| Cellulose hydrolysis | − |
| Indol production | − |
| $H_2S$ production | − |
| Acetoin production | − |
| MR test | − |
| Sulfate reduction | − |
| ( Utilization test ) | |
| Citrate | − |
| Malate | − |
| Maleate | − |
| Malonate | − |
| Propionate | − |
| Gluconate | − |
| Succinate | − |
| ( Acid formation from sugar ) | |
| Adonitol | − |
| L(+)-Arabinose | − |
| Cellobiose | − |
| Dulsitol | − |
| Meso-Erythritol | − |
| Fractose | + |
| Galactose | (+) |

| | |
|---|---|
| Glucose | + |
| Glycerin | (+) |
| Inositol | − |
| Inulin | − |
| Lactose | − |
| Maltose | + |
| Mannitol | − |
| Mannose | (+) |
| Melibiose | − |
| Raffinose | − |
| L(+)-rhamnose | − |
| D-ribose | + |
| Salicin | − |
| L-sorbose | − |
| Sorbitol | − |
| Starch | + |
| Saccharose | + |
| Xylose | (+) |
| Trehalose | + |
| Main production of acid from glucose | Lactic acid, acetic acid |

Media and culture conditions used hereinabove are as follows.

Base medium : Tryptosoy broth ( Difco 0370-01-1 ) added with yeast extract 0.5 %.

Growth temperature : standing culture at 40 °C, 45°C and 52°C.

Growth : shaking culture

Gelatin hydrolysis : basal medium + gelatin 0.4 % + agar 1.5 %, cultured in Petri dish.

BCP milk medium : basal medium + skimmed milk 10 % , BCP 1 % ( 0.2 % aqueous solution ), shaking culture.

Casein hydrolysis : basal medium + skimmed milk 10 % + agar 1.5 %, cultured in Petri dish.

Esculin hydrolysis : basal medium + esculin 1.0 % + agar 1.5 % + ammonium ferrous citrate 1.0 % (10 % aqueous solution), slant cultured.

Cellulose hydrolysis : basal medium with filter paper, shaking culture.

Acetoin production : basal medium + glucose 1.0 %, shaking culture.

MR test : pH measured in medium for acetoin production( MRpH test paper ).

$H_2S$ production : standing culture with basal medium. Detection using lead acetate paper.

Nitrate reduction : basal medium + $NaNO_3$ 0.1 %, shaking culture.

Utilization of organic salt : basal medium + organic salt 0.3 %, shaking culture. pH measured by pH meter.

Acid formation from sugar : sugar 1.0 % added in a medium consisting of Trypton 1.7 %, Soyton 0.3 %, NaCℓ 0.5 % and yeast extract 0.5 % ( pH 7.0 ) shaking culture. pH measured by pH meter.

Urease production : filter sterilized urea added in basal medium, shaking culture.

According to the above taxonomical properties, the novel microroganism strain of the present invention is a Gram positive microaerophilic bacillus, which is catalase negative and has a main production of lactic acid and acetic acid from glucose. Furthermore it is a thermophile which does not grow at 40 °C in the culture conditions hereinbefore but grows at 45°C and 52 °C.

Bergey's Manual of Systematic Bacteriology. Vol. 2, illustrates six genera of spore forming bacteria having the specific features hereinbelow:

① Genus Bacillus : Aerobic or facultative anaerobic, catalase positive bacillus.
② Genus Sporolactobacillus : Microaerophilic, catalase negative bacillus.
③ Genus Clostridium : Anaerobic, catalase negative bacillus.
④ Genus Desulfotomaculum : Anaerobic, catalase negative bacillus.
⑤ Genus Sporosarcina : aerobic, catalase positive coccus.
⑥ Genus Oscillospira : anaerobic, large bacillus.

The present microorganism strain shows weak growth under aerobic conditons and is a microaerophilic and catalase negative bacillus. It thus belongs to the genus Sporolactobacillus. Genus Sporolactobacillus inulinus is the only species described in Bergey's Manual of Systematic Bacteriology, Vol. 2, and no novel species has been found in the International Journal of Systematic Bacteriology up to Vol. 1 in 1988.

The axonomic properties of Sporolactobacillus inulinus (S • inulinus) in comparison with those of the present strain are as follows.

| | S. inulinus | The present strain |
|---|---|---|
| (Acid from glucose) | Mainly lactic acid | lactic acid acetic acid |
| Catalase production | − | − |
| Oxidase production | − | − |
| Nitrate reduction | − | − |
| Indol production | − | − |
| (Acid formation from sugar) | | |
| Fractose | + | + |
| Glucose | + | + |
| Inulin | + | − |
| Maltose | + | + |
| Mannose | + | + |
| Raffinose | + | − |
| Saccharose | + | + |
| Trehalose | + | + |

| | | |
|---|---|---|
| Mannitol | + | − |
| Solbitol | + | − |
| Arabinose | − | − |
| Xylose | − | − |
| Galactose | − | (+) |
| Lactose | − | − |
| Melibiose | − | − |
| Cellobiose | − | − |
| Starch | − | + |
| Glycerin | − | (+) |
| Erythritol | − | − |
| Adonitol | − | − |
| Rhamnose | − | − |
| Salicin | − | − |
| Growth temperature | 15 ~ 40 ℃ | > 45 ℃ |

( +: positive, − : negative, (+) : weak positive )

According to the above comparison, the present strain and S. inulinus have specific differences in growth temperature and in acid formation from glucose. Accordingly the present strain is a novel strain belonging to the genus Sporolactobacillus and has been designated Sporolactobacillus sp. 78-3. This strain was deposited in the Fermentation Research Institute in the permanent culture collection on 9 September 1988 and has been assigned the deposit number FERM BP-2517.

In the process for the production of L-alanine dehydrogenase of the present invention, the L-alanine dehydrogenase producing microorganism belonging to the genus Sporolactobacillus can be used. A preferred example is the aforementioned novel strain microorgonism belonging to genus Sporolactobacillus. The L-alanine dehydrogenase producing microorganism belonging to the genus Sporolactobacillus can be cultured by a conventional culture process for antibiotics or enzyme production. Liquid or solid culture can be applied. In an industrial process, the L-alanine dehydrogenase producing microorganism belonging to the genus Sporolactobacillus is inoculated in a medium and cultured under submerged aeration conditions.

The nutrient sources for the medium are conventional media for microorganism cultivation. Preferable carbon sources are assimilable carbon sources, for example carbohydrate such as glucose, saccharose, lactose, galactose, maltose, mannitol, sorbitol dextrin, molasses or soluble starch, and various organic acids. Examples of nitrogen sources are assimilable nitrogen source such as peptone, powdered yeast extract, meat extract, soybean powder and casein hydrolyzate. Various salts such as those containing phosphate, magnesium, calcium, potassium, sodium, zinc, iron, manganese or halogen, and vitamins are preferably used.

The culture temperature can be varied depending on the growth of the L-alanine dehydrogenase producing microorganisms belonging to the genus Sporolactobacillus and L-alanine dehydrogenase production, and is preferably 45 ~ 60 °C, more preferably about 52°C. The culture time depends on the conditions and is ususally 1 ~ 3 days. Cultivation should be terminated at the stage of maximum production of the enzyme.

The L-alanine dehydrogenase is isolated from the thus obtained culture mass. Example of the enzyme isolation are treatment of the culture medium by filtration centrifugation to separate microbial cells and treatment of the isolated bacterial cells with ultrasonication, French press, mechanical disruption using glass beads or freezing-disruption, or enzymatic digestion with lysozyme to obtain a crude L-alanine dehydrogenase solution. The L-alanine dehydrogenase can be obtained from the crude enzyme solution by applying known means for isolation and purification of proteins and enzymes. For example a fractional precipitation method by adding an organic solvent such as acetone, methanol, ethanol or isopropanol, or salting-out by adding ammonium sulfate, sodium sulfate, potassium phosphate or ammonium aluminium to the crude solution containing L-alanine dehydrogenase can be applied. Furthermore the precipitate can be purified, if necessary, using a molecular sieve, chromatography, electrophoresis or ultracentrifugation. Purification can be performed by using the physicochemical properties of L- alanine dehydrogenase. For example, the enzyme precipitate is dissolved in water or buffer solution, dialysed with a semi-permeable membrane if required, and subjected to ion-exchange chromatography using DEAE-cellulose, DEAE-Sephacel®, DEAE-Sepharose®, DEAE-Sephadex® A-50 (Pharmacia Corp.) or DEAE-Toyopearl® (Toyosoda Co.), or molecular sieving means such as gel-filtration using Sephadex® G-100, G-75 or Sephacryl® S-200. These means can be applied in combination. Purified L-alanine dehydrogenase powder can be obtained by lyophilzation, adding stabilizer, for example a sugar such as mannitol, saccharose or sorbitol, an amino acid such as glutamic acid or glycine, or a protein such as bovine serum albumin. The thus obtained L-alanine dehydrogenase is quite stable in the solution.

The L-alanine dehydrogenase obtained has the following properties.

① Molecular weight 245,000 ± 25,000 [gel-filtration method using polyvinyl-gel (trade name TSK 3000 SW (Toyosoda Co.) column, with moving phase of 50 mM potassium phosphate buffer (pH 6.5) containing 0.2 M NaCℓ]

② Isoelectric point : pH 4.6 ± 0.5 [isoelectric focusing using carrier ampholyte at 4 °C, constant voltage at 700 V for 40 hours]

③ Km-value : 0.84 mM ( pyruvate )
20.0 mM ( $NH_4^+$)
11.0 µM ( NADH )

④ Heat stability The enzyme solution (1.0 U/mℓ) is prepared in 20 mM Tris-HCℓ buffer (pH 8.0), heated for 15 minutes, and remaining enzymatic activity is measured by an enzymatic assay method hereinbelow to obtain the graph shown in Fig. 1. The enzyme is found to be stable at least up to 65 °C.

⑤ Optimum temperature Enzyme activity is measured at each temperature shown in Fig. 2 according to an assay method illustrated hereinbelow by measuring optical absorption at 340 nm for decreasing of NADH. As shown in Fig. 2, the enzyme shows maximum activity at 65°C.

⑥ pH stability : the remaining activity of the enzyme (1.0 U/ mℓ, 40 mM buffer solution) after treating at 80 °C for 15 minutes is determined. As shown in Fig. 3 the enzyme is stable at pH 6.0 ~ 7.5. In the figure ; - Δ - : acetate buffer, pH 5.0 ~ 6.0, - ○ -: phosphate buffer, pH 6.0 ~ 8.0, - ● -: Tris-HCℓ buffer, pH 7.5 ~ 9.0, - □ - : glycine-NaOH buffer, pH 8.5 ~ 9.5.

⑦ Optimum pH approx. pH 9.0 as shown in Fig. 4. The decrease at 340 nm for NADH with 40 mM buffer is measured according to an assay method hereinbelow. In the figure ; - ○ - : phosphate buffer, pH 6.5 ~ 7.5, - ● - : Tris-HCℓ buffer, pH- 7.5 ~ 9.0, - □ -: glycine-NaOH -buffer, pH 9.0 ~ 10.5.

A high potency L-alanine dehydrogenase was obtained by culturing the novel microorganism strain of the present invention, Sporolactobacillus sp. 78-3, and the culture and purification thereof were quite simple and easy.

The enzyme activity is measured by the following assay method.

Reaction mixture :

50 mM triethanolamine
0.3 mM NADH
10 mM pyruvate acid
200 mM $NH_4Cℓ$
( pH 8.5 )

Assay Method :

The reaction mixture (1 mℓ) in a 1 mℓ quarz cell is preincubated at 37°C for 5 minutes. Diluted enzyme solution (0.02 mℓ) with 50 mM triethanolamine buffer (pH 8.5) is added therein and stirred to

initiate the reaction. The decrease of NADH is measured photometrically at 340 nm in the reaction. The enzyme activity is calculated by the following equation on the linear part in the curve.

$$\text{Units/m}\ell = \frac{A}{6.22} \times \frac{1}{T} \times \frac{1.02}{0.02} \times X$$

wherein T: reaction time (min.), X : dilution ratio.

As illustrated hereinbefore, the present invention provides a high efficiency process for production of stable L-alanine dehydrogenase using a novel L-alanine dehydrogenase producing microorganism.

The L-alanine dehydrogenase produced by the process of the present invention can be used for quantitative analysis and assay by applying the following enzymatic reaction:

$$\text{L-alanine} + H_2O + NAD^+ \underset{②}{\overset{①}{\rightleftarrows}} \text{pyruvate} + NH_4^+ + NADH$$

$+ H^+$

The optimum pH of the above oxidative de-aminating reaction ( ① ) is pH 10.5 and that of the reductive aminating reaction( ② ) is pH 9.0.

As clearly seen from the above reaction ① , alanine, a substrate of the L-alanine dehydrogenase obtained by the present invention, can be quantitatively assayed by measuring NADH generation photometrically. Byruvate or ammonium ions can also be assayed by measuring the decrease of NADH. Therefore direct assay of pyruvate or ammonium ions can be performed, and also an enzymatic activity of an enzyme which forms ammonium ions or pyruvate in the previous reaction, or substrates in the said previous reaction, can be assayed. Examples of enzymes which catalyse formation of ammonium ions are :

E C. 3. 5. 1. 1 Asparaginase
E C. 3. 5. 1. 2 Glutaminase
E C. 3. 5. 1. 3 ω-Amidase
E C. 3. 5. 1. 4 Amidase
E C. 3. 5. 1. 5 Urease
E C. 3. 5. 1. 6 β-Ureidopropionase
E C. 3. 5. 1. 7 Unreidopropinase
E C. 3. 5. 1. 12 Biotinidase
E C. 3. 5. 1. 19 Nicotinamidase
E C. 3. 5. 1. 20 Citrullinase
E C. 3. 5. 1. 29 α-( Acetamidometylene ) succinate hydrolase
E C. 3. 5. 1. 30 5-Aminovaleramidase
E C. 3. 5. 1. 35 D-Glutaminase
E C. 3. 5. 1. 38 Glutamin-( asparagin ) ase
E C. 3. 5. 1. 42 Nicotinamidenucleotide amidase
E C. 3. 5. 1. 43 Peptidyl-glutaminase
E C. 3. 5. 1. 44 Glutaminyl-peptide glutaminase
E C. 3. 5. 1. 45 Urease
E C. 3. 5. 3. 5 Formiainoasparartate deiminase
E C. 3. 5. 3. 6 Arginine deiminase
E C. 3. 5. 3. 7 Guanidinobu tyrase
E C. 3. 5. 3. 9 Allantoate deiminase
E C. 3. 5. 4. 1 Cytosine deaminase
E C. 3. 5. 4. 2 Adenine deaminase
E C. 3. 5. 4. 3 Guanine deaminase
E C. 3. 5. 4. 4 Adenosine deaminase

E C. 3. 5. 4. 5 Cytidine deaminase
E C. 3. 5. 4. 6 AMP deaminase
E C. 3. 5. 4. 7 ADP deaminase
E C. 3. 5. 4. 8 Aminoimidazolase
E C. 3. 5. 4. 11 Pterin deaminase
E C. 3. 5. 4. 12 dCMP deamindse
E C. 3. 5. 4. 13 dCTP deaminase
E C. 3. 5. 4. 14 Deoxycytidine deaminase
E C. 3. 5. 4. 15 Guanosine deaminase
E C. 3. 5. 4. 17 Adenosine ( Phosphate ) deaminase
E C. 3. 5. 4. 18 ATP deaminase
E C. 3. 5. 4. 20 Pyrithiamin deaminase
E C. 3. 5. 4. 21 Creatinine deaminase
E C. 3. 5. 4. 22 1-Pyrroline 4-hydroxy 2-carboxylate deaminase
E C. 3. 5. 4. 23 Blasticidin-S deaminase
E C. 3. 5. 4. 24 Sepiapterin deaminase
E C. 3. 5. 5. 1 Nitrilase
E C. 3. 5. 5. 2 Ricinine nitrilase
E C. 3. 5. 5. 3 Cyanate hydorolase

For example EC. 3. 5. 4. 4 adenosine deaminase is assayed in the enzymatic reactions hereinbelow illustrated, by the method of Chaney & Marbach for ammonium ions, which are introduced into a glutamate dehydrogenase (EC. 1. 4. 1. 3) reaction to measure the decrease of NADH. In that reaction, ammonium ions generated therefrom can be introduced into the enzymatic reaction of L-alanine dehydrogenase obtained by the present invention and the said adenosine deaminase can be assayed by measuring photometrically the decrease of NADH at 340 nm.

(1) adenosine + $H_2O$ → inosine + $NH_4^+$
(2) pyruvate + $NH_4^+$ + NADH + $H^+$ → L-alanine + $H_2O$ + $NAD^+$

The enzymatic activity of an enzyme which generates urea can be assayed by the reaction of urease on the generated urea. The thus formed ammonium ions are introduced into the L-alanine dehydrogenase system for direct measurement.

Examples of enzymes which form urea in the enzymatic reaction are :

E C. 3. 5. 2. 1 Barbiturase
E C. 3. 5. 3. 1 Arginase
E C. 3. 5. 3. 2 Glycocyaminase
E C. 3. 5. 3. 3 Creatinase
E C. 3. 5. 3. 4 Allantoicase
E C. 3. 5. 3. 7 Guanidinobutyrase
E C. 3. 5. 3. 10 D-Arginase
E C. 3. 5. 3. 11 Agmatinase
E C. 3. 5. 3. 14 Amidinoaspartase

For example in an assay of EC. 3. 5. 3. 1 arginase, urea generated in the following enzymatic reaction is treated with urease and ammonium ions thus formed are introduced into the enzymatic reaction of L-alanine dehydrogenase and measured photometrically by the decrease of NADH at 340 nm.

(1) arginine + $H_2O$ → lysine + urea
(2) ATP + urea + $H_2O$ → ADP + pi + $CO_2$ + $2NH_4^+$
(3) pyruvate + $NH_4^+$ + NADH + $H^+$ → L-alanine + $H_2O$ + $NAD^+$

As illustrated hereinabove, various analyses and assays can be made using L-alanine dehydrogenase.

## Examples

The following Examples further illustrate the present invention.

Example 1

Culturing Sporolactobacilius sp. 78-3 :

( i )

| | |
|---|---|
| peptone ( Kyokuto Seiyaku So.) | 1.0 % |
| yeast extract ( Kyokuto Seiyaku Co.) | 0.5 % |
| glucose | 1.0 % |
| alanine | 2.0 % |
| $KH_2PO_4$, | 0.3 % |
| $MgSO_4 \cdot 7 H_2O$ | 0.05 % |
| $CaCl_2 \cdot 2 H_2O$ | 0.02 % |
| ( pH 7.0 ) | |

A liquid medium (100 ml) comprising the above composition in a 500 ml Erlenmeyer flask was sterilized at 120 °C for 20 minutes. One loopful of Sporolactobacillus sp. 78-3 was inoculated in the medium and cultured at 50 °C at 120 rpm for 24 hours to obtained a cultured mass (100 ml) ( relative activity 50 U/ml ).

(ii)

| | |
|---|---|
| polypeptone ( Takeda Chem. Ind. ) | 1.0 % |
| yeast extract ( Kyokuto Seiyaku Co.) | 0.2 % |
| soluble starch | 1.0 % |
| alanine | 2.0 % |
| $KH_2PO_4$ | 0.3 % |
| $MgSO_4 \cdot 7 H_2O$ | 0.05 % |
| $CaCl_2 \cdot 2 H_2O$ | 0.02 % |
| ( pH 7.0 ) | |

A liquid medium(20 ℓ) comprising the above composition in a 30 ℓ jar-fermenter was sterilized by heating. Precultured seed culture obtained in step ( i ) hereinabove 100 mℓ was inoculated therein and cultured at 50°C, aeration 20 ℓ/min., inner pressure 0.2 kg/ cm and agitation at 120 rpm for 16 hours to obtain the cultured mass (19 ℓ).

Example 2

Purification of enzyme :

Bacterial cells collected from the cultured broth (19 ℓ) obtained in Example 1, Culture (ii) by centrifugation, suspended in 40 mM phosphate buffer (pH 7.0), were mixed with O.1 % lysozyme 5 ℓ, and solubilized at 37 °C for 30 minutes. The mixture was then adjusted to pH 7.0 and treated at 70 °C for 30 minutes. After immediatly cooling the mixture, the precipitate was centrifugally removed to obtain supernatant solution 4500 mℓ ( enzyme activity 304 U/mℓ). An equal amount of acetate was added to the supernatant solution, mixed well with stirring, then centrifuged to obtained a precipitate. 40 mM phosphate buffer ( pH 7.0 ) (1 ℓ) was added to the precipitate, stirred, and the sediment was removed by centrifugation to obtain a supernatant solution. The supernatant solution was passed through a column of DEAE-Sepharose® CL-6B (Pharmacia Corp.) (500 mℓ). The enzyme solution was eluted with 40 mM phosphate buffer (pH 7.0) (500 mℓ) containing 0.2 M KCℓ, then ammonium sulfate (156 g) was added therein, stirred and centrifuged to obtain a precipitate. Ammonium sulfate (35g) was added to a solution of the said precipitate dissolved in 40 mM phosphate buffer solution (pH 7.0) (200 mℓ), up to ammonium sulfate 30 % saturation, which was passed through a column of Octyl-sepharose® CL-4B (Pharmacia Corp.) (100 mℓ) bufferized with 30 % saturated ammonium sulfate. The column was washed with the same buffer solution (500 mℓ), then passed through the 20 % saturated ammonium sulfate (200 mℓ) to obtain the enzyme solution. The thus obtained enzyme solution was dialyzed twice against 40 mM phosphate buffer solution (pH 7.0 ) (10 ℓ) overnight to yield an enzyme solution (250 mℓ) (enzyme activity : 2750 U/mℓ ,

recovery : 50.3 %).

Referential example 1

Assay of ammonium ions using L-alanine dehydrogenase:

| Reaction mixture : | |
|---|---|
| Tris-HCℓ buffer (pH 8.5) | 50 mM |
| NADH (Oriental yeast Co.) | 0.2 mM |
| Sodium pyruvate | 10 mM |
| L-alanine dehydrogenase | 3.75 U |

10 mM $NH_4Cℓ$ was added to the above reaction mixture 1 mℓ. In Fig. 5; - □ - : 1 μℓ of 10 mM $NH_4Cℓ$, - Δ -: 2.5 μℓ , - ● -: 5 μℓ and - ○ - : 10 μℓ , respectively. The decrease in optical density at 340 nm was measured. The decrease in optical density at 340 nm within one minute between the 7th and 8th minutes is shown in Fig. 6., in which linearity crossing original point was obtained.

Referential example 2

Assay of urea using L-alanine dehydrogenase:

| Reaction mixture : | |
|---|---|
| Tris-HCℓ buffer (pH 8.5) | 50 mM |
| NADH (Oriental Yeast Co.) | 0.2 mM |
| Sodium pyruvate | 10 mM |
| L-alanine dehydrogenase | 5.0 U |
| Urease (jack beans) | 25 U |

4 μℓ, 8 μℓ, 12 μℓ, 20 μℓ and 40 μℓ, respectively of urea solution (50 mg/ dℓ ) was each added to the reaction mixture (3 mℓ) hereinabove. The decrease in optical density at 340 nm was measured. The decrease within one minute between the 7th and 8th minutes is shown in Fig. 7, wherein a linearity crossing at original point is obtained.

Referencial example 3

Measurement of adenosine deaminase activity using L-alanine dehydrogenase:

Reaction mixture :

| | |
|---|---|
| Tris-HCℓ buffer (pH 8.5) | 50 mM |
| NADH (Oriental Yeast Co.) | 0.2 mM |
| Sodium pyruvate | 10 mM |
| L-alanine dehydrogenase | 20.0 U |

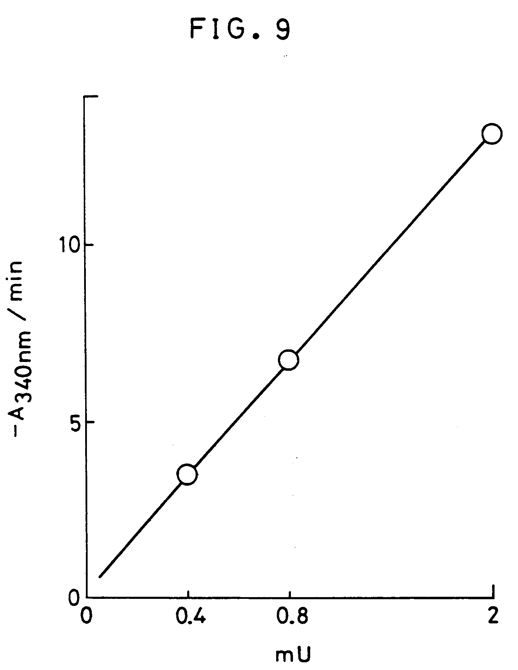

Adenosine deaminase, each 0.4 mU, 0.8 mU and 2 mU, respectively, was added to the above reaction mixture (1 mℓ) and incubated at 37 °C for 10 minutes. 1 mM adenosine (100 μℓ) was added to the reaction mixture, and the decrease in optical density at 340 nm was measured. The result is shown in Fig. 8. In Fig. 9, a decrease at 340 nm within 1 minute between the 16th to 17th minutes is shown. Good linearity is obtained.

In the assay systems hereinabove illustrated, the pH of the reaction buffer is pH 8 ~ 10, prefereably 8.5 ~ 9.5. The concentration of NADH is 0.01 mM ~ 0.6 mM, preferably 0.2 ~ 0.3 mM.

The concentration of pyruvate is 0.5 mM ~ 0.5 M, preferably 5 ~ 20 mM.

The concentration of L-alanine dehydrogenase is 0.1 ~500 U/ mℓ, preferably 10 ~ 30 U/ mℓ.

If required a lactic acid dehydrogenase (LDH) inhibitor such as oxaminic acid and oxalic acid in an amount of 10 ~ 70 mM was added for prevention of the effects of serum LDH.

Brief description of the drawings

Fig. 1 : Heat stability of L-alanine dehydrogenase,
Fig. 2 : Optimum temperature of L-alanine dehydrogenase,
Fig. 3 : pH-stability of L-alanine dehydrogenase,
Fig. 4 : Optimum pH of L-alanine dehydrogenase,
Fig. 5 : Rate assay of ammonium ion by altering $NH_4C\ell$ concentration,
Fig. 6 : Assay curve of ammonium ion,
Fig. 7 : Assay curve of urea,
Fig. 8 : Rate assay of adenosine deaminase by altering concentration of adenosine deaminase ; and
Fig. 9 : Standard curve of adenosine deaminase.

**Claims**

1. A process for the production of L-alanine dehydrogenase, which comprises culturing a L-alanine dehydrogenase-producing microorganism belonging to genus Sporolactobacillus in a nutrient medium and isolating L-alanine dehydrogenase from the cultured mass.

2. A process according to claim 1 wherein the microorganism is Sporolactobacillus sp. 78-3 (FERM BP-2517) or a mutant or variant thereof having the ability to produce L-alanine dehydrogenase.

3. A process according to claim 1 or 2 which additionally comprises using the L-alanine dehydrogenase obtained to catalyse the reaction (I):

$$\text{L-alanine} + H_2O + NAD^+ \leftrightarrows \text{pyruvate} + NH_4^+ + NADH + H^+$$

4. A microorganism strain belonging to the genus Sporolactobacillus which is a thermophile which cannot grow at 40 ° C and can grow at 45 ° C and 52 ° C.

5. A strain according to claim 4 which can assimilate glucose and produce lactic acid and acetic acid.

6. A strain according to claim 4 or 5 having the following taxonomical properties:
[ + : positive, -: negative, ( + ): weakly positive]
① Morphological properties :

| | |
|---|---|
| Form | bacillus |
| Mobility | + |
| Gram-stain | + |
| Peritrichal | + |
| Spore | + |
| Acid fastness | - |

Tadpole-like cells appear at 15~20 hours culture.
② Growth on various media:
i) Nutrient agar slant medium :
Growth : weak, filiform.
Color : transparent, ocherous.
Soluble pigment : no production.
ii) Nutrient agar plate medium :
Growth : weak, round and flat ~ convex smooth and small colony.
Color : transparent, ocherous.
Soluble pigment : no production.
iii) Liquid medium :
Weak growth uniformly turbid.

vi) BCP milk medium :
No change.

③ Biochemical properties :

| | |
|---|---|
| Aerobic growth | (+) |
| Anaerobic growth | - |
| Catalase production | - |

Lactic acid and acetic acid formation from glucose.

④ Acid formulation from sugar :

| | |
|---|---|
| Inulin | - |
| Raffinose | - |
| Mannitol | - |
| Sorbitol | - |
| Galactose | (+) |
| Starch | + |
| Glycerin | (+) |

**7.** A strain according to any one of claims 4 to 6, which has the ability to produce L-alanine dehydrogenase.

**8.** A strain according to claim 7, which is Sporolactobacillus sp. 78-3 (FERM BP-2517) or a mutant or variant thereof having the ability to produce L-alanine dehydrogenase.

**9.** A culture of a strain as defined in any one of claims 4 to 8 in a culture medium containing an assimilable source of carbon, an assimilable source of nitrogen and, if desired, vitamins and inorganic salts other than any such salts which may be used as a nitrogen source, and substantially free from other microorganisms.

**Patentansprüche**

**1.** Verfahren zur Herstellung von L-Alanin-Dehydrogenase, das das Kultivieren eines L-Alanin-Dehydrogenase erzeugenden Mikroorganismus, der zum Genus Sporolactobacillus gehört, in einem Nährmedium und Isolieren von L-Alanin-Dehydrogenase aus der Kulturmasse umfaßt.

**2.** Verfahren nach Anspruch 1, worin der Mikroorganismus Sporolactobacillus sp. 78-3 (FERM BP-2517) oder eine Mutante davon oder eine Variante davon mit der Fähigkeit zur Produktion von L-Alanin-Dehydrogenase ist.

**3.** Verfahren nach Anspruch 1 oder 2, welches außerdem die Verwendung der erhaltenen L-Alanin-Dehydrogenase zum Katalysieren der Reaktion (I) umfaßt:

$$\text{L-Alanin} + H_2O + NAD^+ \leftrightarrows \text{Pyruvat} + NH_4^+ + NADH + H^+ \qquad (I)$$

**4.** Mikroorganismusstamm, der zum Genus Sporolactobacillus gehört, der ein thermophiler Mikroorganismus ist, der nicht bei 40 °C wachsen kann und bei 45 °C und bei 52 °C wachsen kann.

**5.** Stamm nach Anspruch 4, welcher Glucose aufnehmen (assimilieren) kann und Milchsäure und Essigsäure produzieren kann.

**6.** Stamm nach Anspruch 4 oder 5, welcher die folgenden taxonomischen Eigenschaften aufweist:
(+: positiv; -: negativ; (+): schwach positiv)

(1) Morphologische Eigenschaften:

| | |
|---|---|
| Form: | Bacillus |
| Mobilität: | + |
| Gram-Färbung: | + |
| Peritrichen: | + |
| Sporen: | + |
| Säurebeständigkeit: | - |

Kaulquappenartige Zellen erscheinen bei einer Kulturzeit von 15 bis 20 h.

(2): Wachstum auf verschiedenen Medien:

(i) Nähragar-Schrägmedium:
Wachstum: schwach, fadenförmig;
Farbe: transparent, ockerfarbig;
Lösliches Pigment: keine Produktion.

(ii) Nähragar-Plattenmedium:
Wachstum: schwach, rund und flach bis konvex glatt und kleine Kolonie;
Farbe: transparent, ockerfarbig;
Lösliches Pigment: keine Produktion.

(iii) Flüssiges Medium:
Schwaches Wachstum, einheitlich trübe.

(iv) BCP-Milch-Medium:
Keine Änderung.

(3) Biochemische Eigenschaften:

| | |
|---|---|
| Aerobes Wachstum | (+) |
| Anaerobes Wachstum | - |
| Katalaseproduktion | - |

Bildung von Milchsäure und Essigsäure aus Glucose.

(4) Säurebildung aus Zucker:

| | |
|---|---|
| Inulin | - |
| Raffinose | - |
| Mannitol | - |
| Sorbitol | - |
| Galactose | (+) |
| Stärke | + |
| Glycerin | (+) |

**7.** Stamm nach einem der Ansprüche 4 bis 6, welcher die Fähigkeit aufweist, L-Alanin-Dehydrogenase zu produzieren.

**8.** Stamm nach Anspruch 7, nämlich Sporolactobacillus sp. 78-3 (FERM BP-2517) oder eine Mutante davon oder eine Variante davon mit der Fähigkeit zur Produktion von L-Alanin-Dehydrogenase.

**9.** Kultur eines Stamms, wie er in einem der Ansprüche 4 bis 8 definiert wurde, in einem Kulturmedium, das eine assimilierbare Kohlenstoffquelle, eine assimilierbare Stickstoffquelle und - sofern erwünscht - Vitamine und anorganische Salze enthält, die andere Salze sind als irgendwelche derartigen Salze, die als Stickstoffquelle verwendet werden können, und das im wesentlichen frei ist von anderen Mikroorganismen.

## Revendications

**1.** Procédé de préparation de L-alanine déshydrogénase qui comprend la mise en culture d'un microorganisme producteur de L-alanine, déshydrogénase appartenant au genre Sporolactobacillus, dans un

milieu nutritif, et l'isolement de la L-alanine déshydrogénase de la masse de culture.

**2.** Procédé selon la revendication 1, où le microorganisme est Sporolactobacillus sp. 78-3 (FERM BP-2517) ou bien son mutant ou sa variante ayant l'aptitude de produire la L-alanine déshydrogénase.

**3.** Procédé selon la revendication 1 ou 2, qui comporte de plus l'utilisation de la L-alanine déshydrogénase obtenue pour catalyser la réaction (I) :

L-alanine + $H_2O$ + $NAD^+$ $\rightleftarrows$ pyruvate + $NH_4^+$ + NADH + $H^+$

**4.** Souche de microorganisme appartenant au genre Sporolactobacillus qui est un thermophile qui ne peut croître à 40 °C et peut croître à 45 °C et à 52 °C.

**5.** Souche selon la revendication 4, qui peut assimiler le glucose et produire l'acide lactique et l'acide acétique.

**6.** Souche selon la revendication 4 ou 5, ayant les propriétés taxonomiques suivantes :
(+ : positif, - : négatif, (+) : faiblement positif)
① Propriétés morphologiques :

| | |
|---|---|
| Forme | bacille |
| Mobilité | + |
| Coloration Gram | + |
| Péritriche | + |
| Spore | + |
| Solidité aux acides | - |

Cellules ressemblant à des têtards apparaissent à 15 20 heures de culture.
② Croissance sur divers milieux :
i) Milieu oblique agar nutritif :
Croissance : faible, filiforme.
Couleur : transparente, ocreuse.
Pigment soluble : pas de production.
(ii) Milieu plat d'agar nutritif :
Croissance : faible, petite colonie lisse ronde et plate à convexe.
Couleur : transparente, ocreuse.
Pigment soluble : pas de production.
(iii) Milieu liquide :
Faible croissance, uniformément turbide.
(iv) Milieu lait BCP :
Pas de changement.
③ Propriétés biochimiques :

| | |
|---|---|
| Croissance aérobie | (+) |
| Croissance anaérobie | - |
| Production de catalase | - |

Formation d'acide lactique et d'acide acétique à partir du glucose.
④ Formation d'acides à partir de sucre :

| | |
|---|---|
| Inuline | - |
| Raffinose | - |
| Mannitol | - |
| Sorbitol | - |
| Galactose | (+) |
| Amidon | + |
| Glycérine | (+) |

**7.** Souche selon l'une quelconque des revendications 4 à 6, qui a l'aptitude de produire la L-alanine déshydrogénase.

**8.** Souche selon la revendication 7, qui est Sporolactobacillus sp. 78-3 (FERM BP-2517) ou son mutant et sa variante ayant l'aptitude de produire la L-alanine déshydrogénase.

**9.** Culture d'une souche telle que définie selon l'une quelconque des revendications 4 à 8 dans un milieu de culture contenant une source assimilable de carbone, une source assimilable d'azote et,si on le souhaite, des vitamines et des sels inorganiques autres que les sels qui peuvent être utilisés comme source d' azote et sensiblement sans autre microorganisme.

FIG. 1

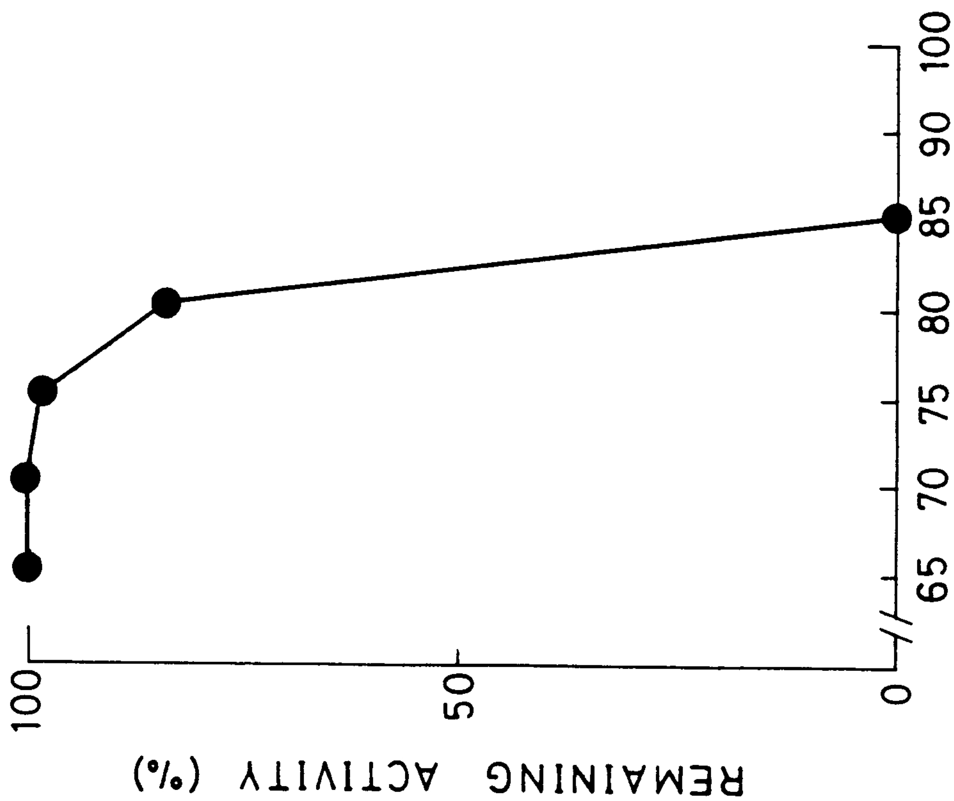
REMAINING ACTIVITY (%)
100
50
0
65
70
75
80
85
90
100
°C

FIG. 2

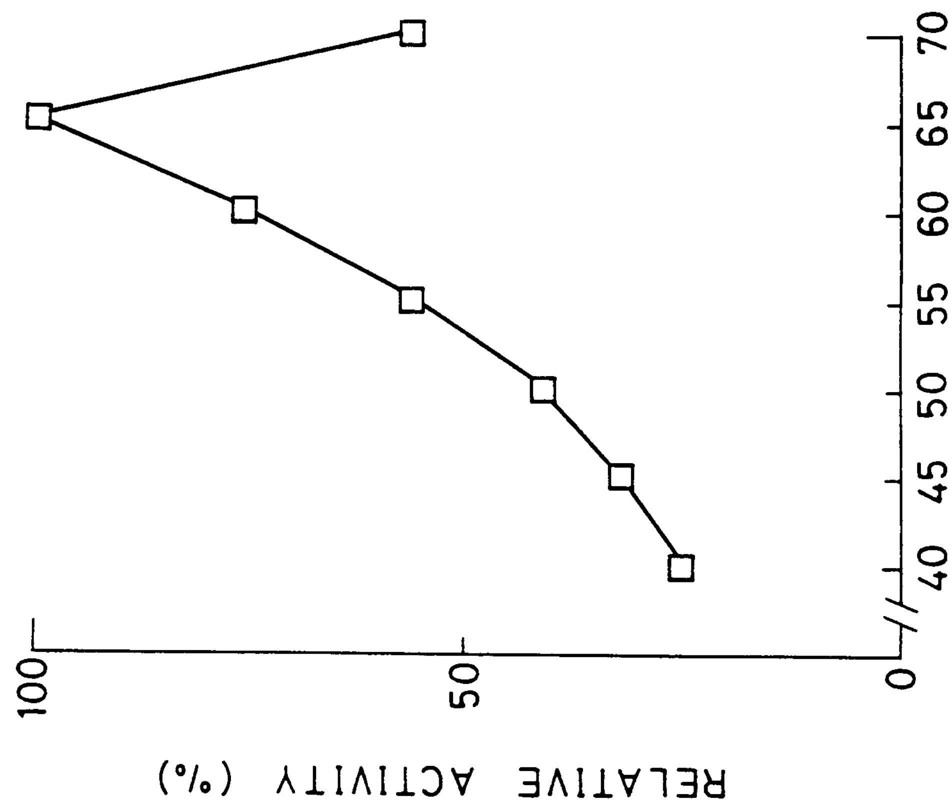
RELATIVE ACTIVITY (%)
100
50
0
40
45
50
55
60
65
70
°C

FIG. 4

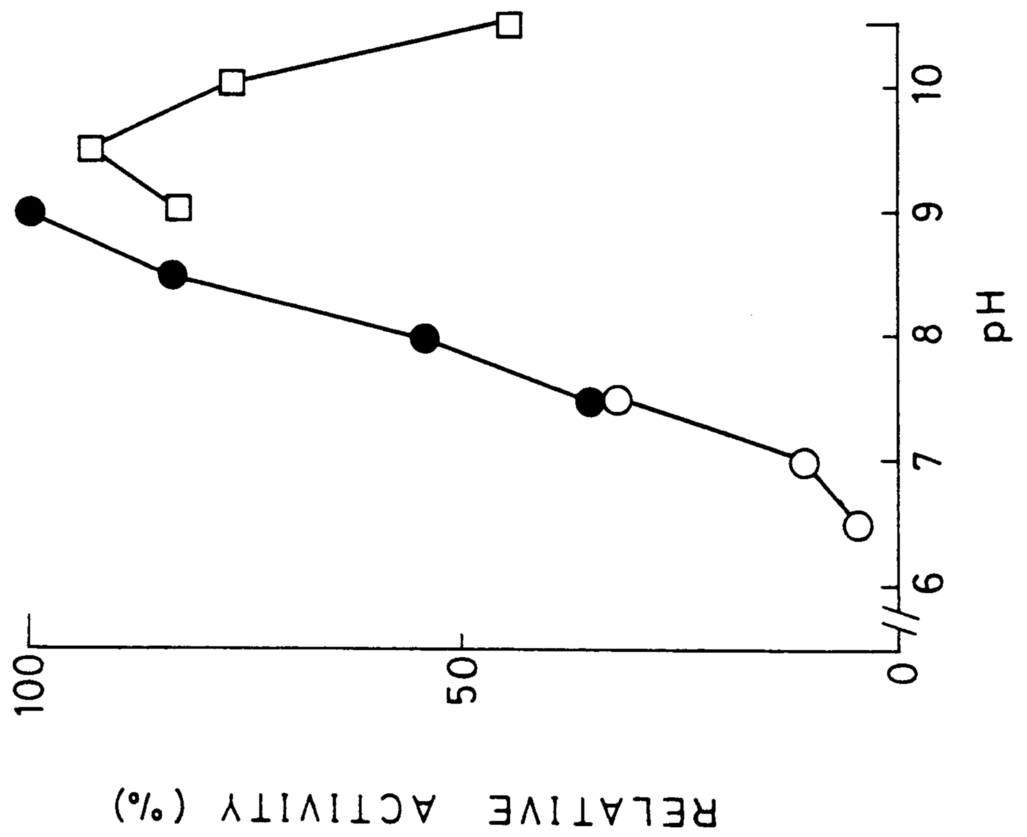
RELATIVE ACTIVITY (%)
100
50
0
pH
6
7
8
9
10

FIG. 3

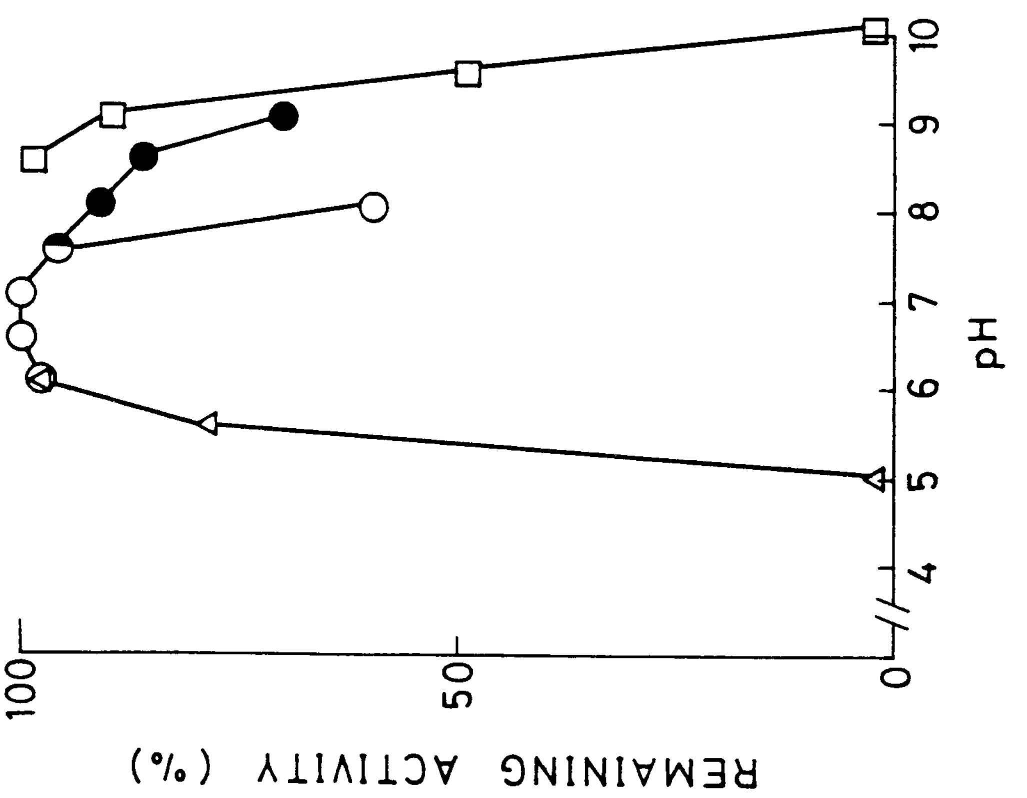
REMAINING ACTIVITY (%)
100
50
0
pH
4
5
6
7
8
9
10

FIG. 5

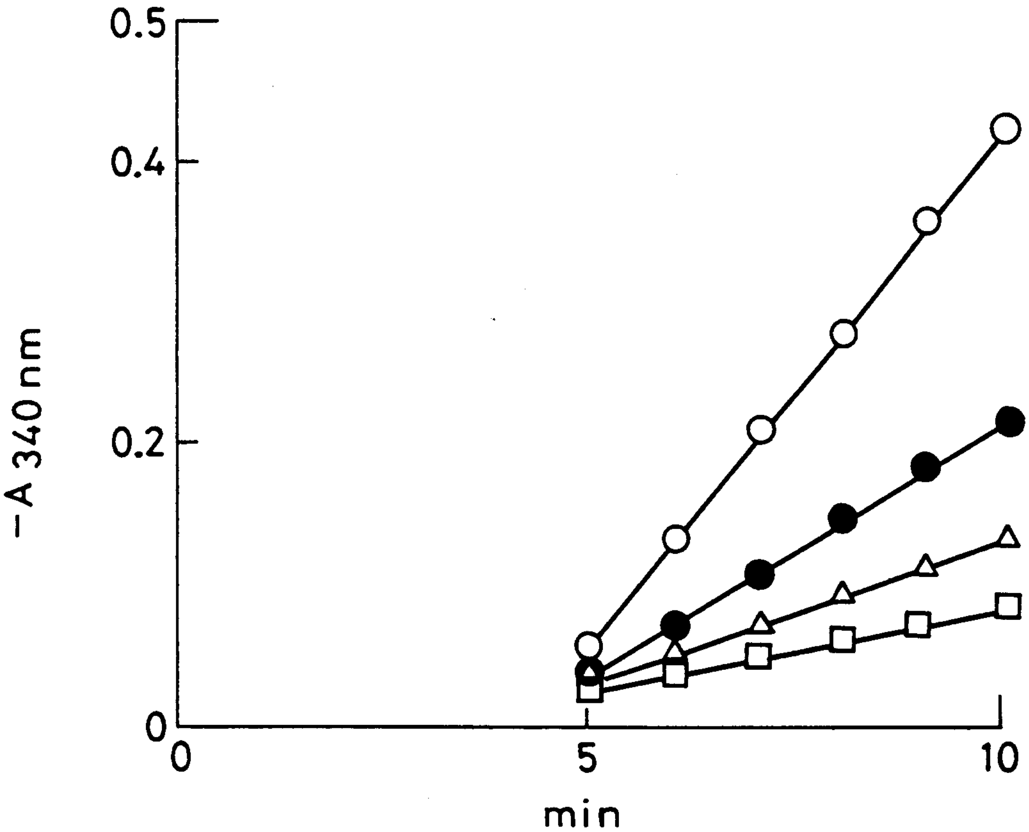


FIG. 6

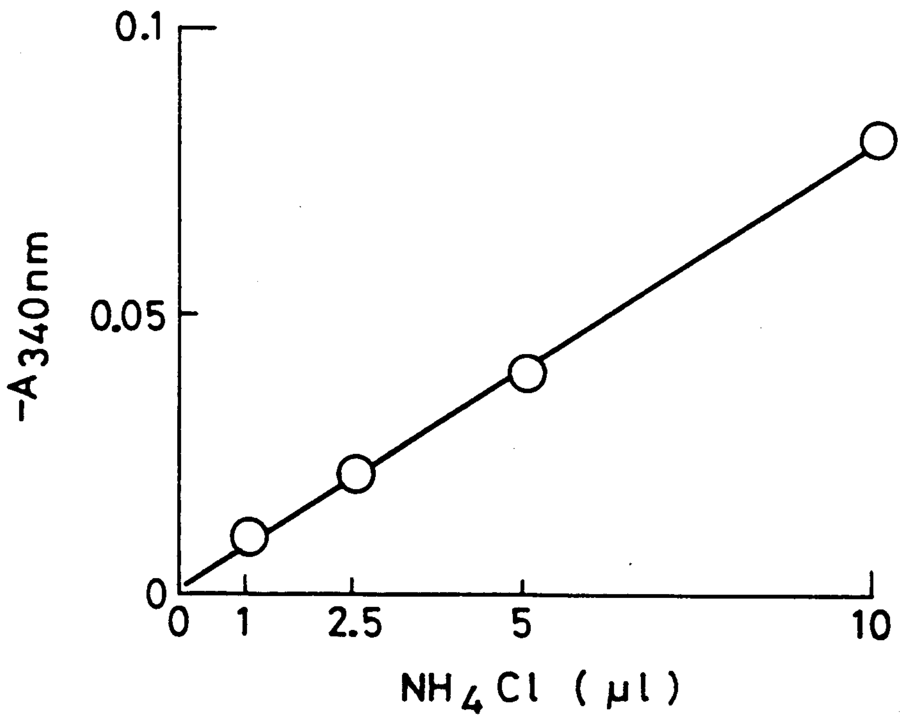

FIG. 7

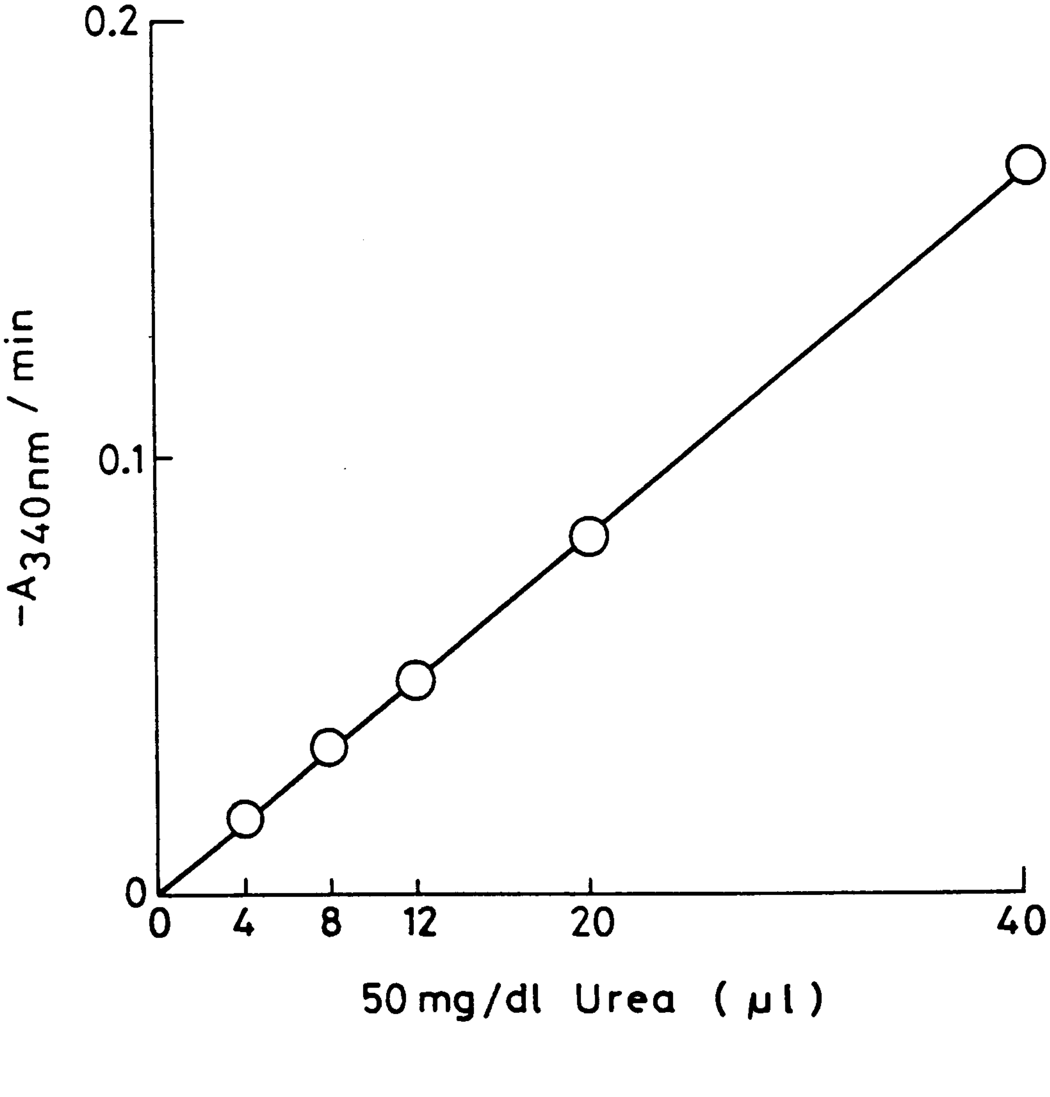

FIG. 8

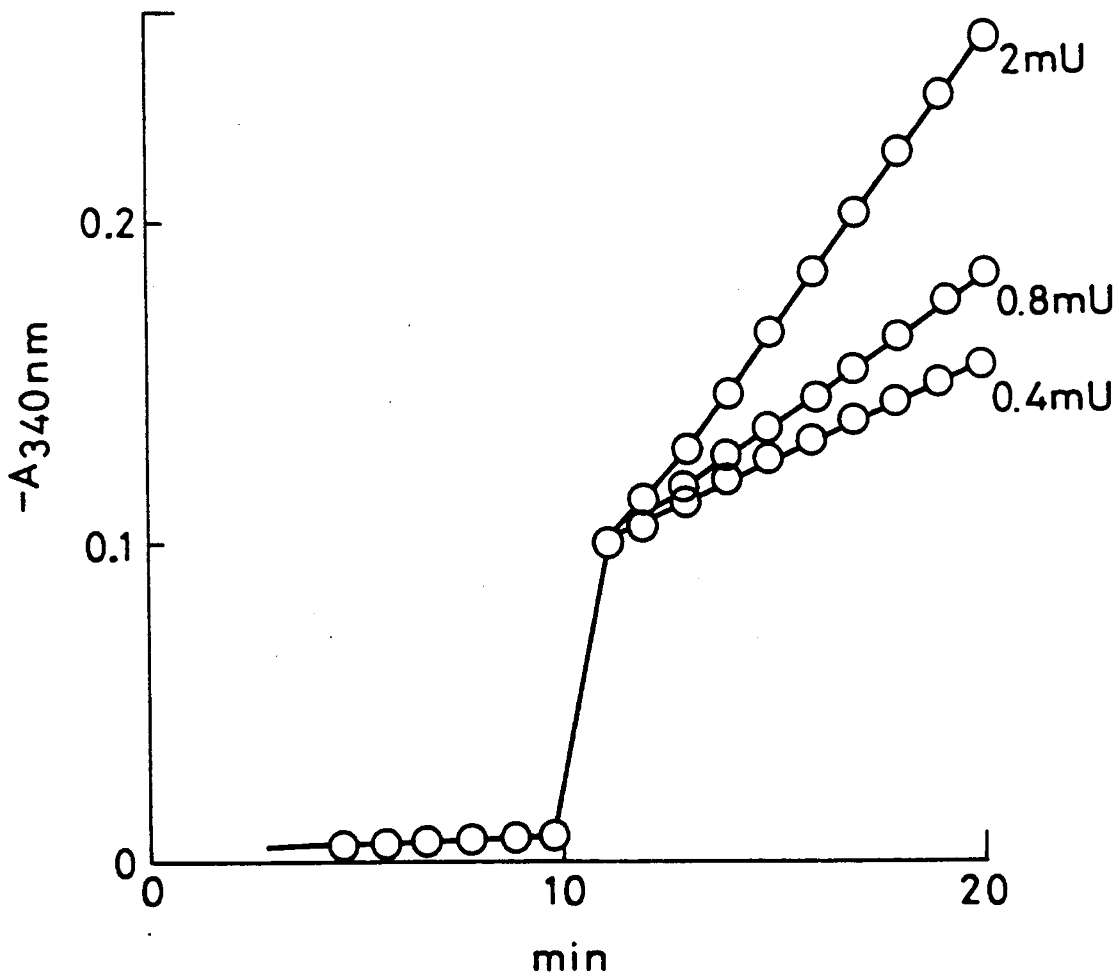

FIG. 9